# FASCICULE DE BREVET EUROPEEN

(11) **EP 1 328 235 B1**
(45) Date de publication et mention de la délivrance du brevet: **24.01.2007**
(21) Numéro de dépôt: 01921471.7
(22) Date de dépôt: 04.04.2001
(51) Int. Cl.: A61K 8/27, A61K 8/29, A61K 8/897, A61Q 17/04

(54) **LAIT ECRAN SOLAIRE**
SONNENSCHUTZMILCH
SUNSCREEN MILK

(30) Priorité: 05.04.2000 FR 0004360
(43) Date de publication de la demande: 23.07.2003
(73) Titulaire: Laboratoires Expanscience, 92400 Courbevoie (FR)
(72) Inventeur: CHOULOT, Jean-Christophe, F-78120 Rambouillet (FR); PETIT, Nathalie, F-28130 Saint-Martin-de-Nigelles (FR); MSIKA, Philippe, F-75018 Paris (FR); FERRARIS, Patricia, F-78125 Raizeux (FR); RIBES, Bernard, F-28100 Dreux (FR)
(74) Mandataire: Warcoin, Jacques
(86) Numéro de dépôt international: PCT/FR2001/001007
(87) Numéro de publication internationale: WO 2001/074294

(56) Documents cités:
- EP-A- 0 832 644
- EP-A- 1 016 400
- FR-A- 2 731 615
- US-A- 5 428 142
- US-A- 5 831 080
- DATABASE CAPLUS [en ligne] retrieved from STN Database accession no. 1995:524043 XP002153307 & JP 07 041416 A (SHISEIDO) 10 février 1995 (1995-02-10)

## Description

La présente invention se rapporte à une composition cosmétique ou dermatologique à base d'agent écran minéral, notamment pour l'enfant, présentant à la fois une très bonne fluidité et une excellente photo-protection à spectre large. On sait depuis longtemps que les rayons ultraviolets (UV) émis par le soleil ont non seulement des effets visibles à courts termes (coups de soleil, bronzage) mais provoquent aussi des dégâts à longs termes, en particulier des cancers cutanés. Des écrans solaires adaptés ont été mis au point avec pour cible particulière les enfants, dans la mesure où il a été montré que l'enfance est la période clé en termes de mémorisation des dégâts induits par une photoprotection incomplète.

Historiquement, les premiers photoprotecteurs à avoir été utilisés ont été des filtres de synthèse, solubles dans l'huile ou dans l'eau. Ces filtres de synthèse (organiques) ont la propriété d'absorber les rayons UV à la place de la peau mais présentent un certain nombre d'inconvénients, en particulier un indice de protection qui diminue avec le temps et l'ensoleillement (photo-instabilité), une pénétration à travers la peau qui pose la question de leur devenir dans l'organisme (notamment chez l'enfant plus sensible aux effets toxiques) et enfin une absorption des UVB ou UVA mais trop rarement des deux en même temps. Ces dernières années, on a utilisé des agents écrans minéraux (ou "inorganiques") notamment pour des produits solaires pour les enfants. Généralement, il s'agit de dioxyde de titane et d'oxyde de zinc qui ont l'aspect d'une poudre blanche constituée de petites particules de ces pigments. Si ces produits déjà connus présentent certains avantages, en particulier une absence de pénétration à travers la peau, une stabilité au soleil et dans le temps ainsi qu'une totale inertie vis-à-vis des réactions cutanées (irritations, allergies, etc), il restait jusqu'à présent un inconvénient majeur pour l'utilisateur, à savoir un aspect couvrant très blanc et une difficulté à étaler, empêchant l'obtention d'une protection efficace dans la mesure où soit l'utilisateur ne parvenait pas à appliquer correctement le produit sur toutes les zones à protéger soit il ne cherchait même pas à obtenir une telle application, compte tenu de l'aspect peu esthétique et du caractère peu pratique de ces produits couvrants très blanc et pâteux, d'où une photo-protection incomplète des zones à protéger en priorité, c'est à dire le visage, le cou, les épaules, les mains et les pieds. En fait, la viscosité importante des produits existants résulte d'un problème de dispersion non homogène des pigments minéraux dans les excipients connus. En effet, une dispersion non homogène empêchant non seulement l'obtention d'une bonne photo-protection (indice de protection élevé) mais aussi d'une photo-protection à spectre large (UVB, UVA courts et UVA longs) même dans les zones où l'application du produit peut sembler correcte à l'utilisateur, il était nécessaire jusqu'à ce jour de compenser ce manque d'homogénéité en augmentant la quantité de pigment minéral, donc la viscosité du produit. Une solution insatisfaisante pour concilier photo-protection à spectre large et viscosité moins importante consistait en particulier à diminuer la charge en pigment minéral en la remplaçant par des filtres organiques dont la présence est néanmoins à éviter notamment dans des produits pour enfants comme expliqué ci-dessus. On a maintenant constaté de manière tout à fait surprenante et inattendue que l'association de certains écrans minéraux avec certains agents émulsionnants permet d'obtenir une composition cosmétique ou dermatologique présentant à la fois une très bonne fluidité et une excellente photo-protection qui plus est à spectre large, en l'absence de la présence impérative de filtres organiques. La composition cosmétique ou dermatologique selon l'invention fournit ainsi tout d'abord une protection de surface spectaculaire, se traduisant par des indices de protection pouvant être supérieurs à 70, comme illustré par les exemples ci-après, tout en présentant une très bonne fluidité, c'est à dire une viscosité pouvant être inférieure à 100 Pa.s (100 000 centipoises) à 25°C.

En outre, non seulement cette composition cosmétique ou dermatologique n'a plus l'inconvénient d'un aspect visqueux voire pâteux mais, aussi, elle présente une transparence totale (aucun effet blanchissant), avantage déterminant sur le plan cosmétique pour un produit solaire à haute protection.

La présente invention a ainsi pour objet une composition cosmétique ou dermatologique pour la protection contre les rayons ultraviolets, à base d'agent écran inorganique, caractérisée en ce qu'elle comprend une émulsion eau-dans-huile contenant une association d'un agent écran inorganique particulaire, constitué par un mélange de dioxyde de titane dopé en fer ou de dioxyde de titane hydrophobe et d'oxyde de zinc, et d'au moins un agent émulsionnant choisi dans le groupe constitué par les dérivés siliconés à composante glucosique comprenant un nombre de motifs glucose compris entre 2 et 10, l'agent écran inorganique particulaire étant dispersé de manière homogène dans l'émulsion eau-dans-huile et sa taille moyenne de particule étant comprise entre 1 et 100 nanomètres, et l'agent écran inorganique particulaire étant présent à hauteur de 6 à 40 % en poids.

Au sens de la présente invention, on entend par dérivés siliconés à composante glucosique, tous dérivés siliconés comprenant un nombre de motifs glucose compris entre 2 et 10. On préfère, à titre de dérivé siliconé, les (C₂-C₃₀)alkylsilicones et les amino(C₂-C₃₀)alkylsilicones. Ainsi, parmi les dérivés siliconés à composante glucosique selon la présente invention, on peut notamment citer les dérivés obtenus par la réaction des polymères de diméthicone avec des polymères de glucose. Comme polymères de diméthicone, on peut citer à titre d'exemples, l'amino bispropyl diméthicone, l'aminopropyl diméthicone, l'amodiméthicone, la cétyldiméthicone, l'hexyldiméthicone, l'octyl diméthicone et la stéaryl diméthicone.

Dans un mode particulier de réalisation de la composition selon l'invention, le dérivé siliconé à composante glucosique est le produit de la réaction de l'octyldiméthicone avec un polymère de glucose, appelé éthylhexyl diméthicone éthoxy glucoside (dénomination INCI, ethylhexyl dimethicone ethoxy glucoside n° 528 in International Cosmetic Ingredients Dictionary and Handbook, 8^{ème} édition).

Selon la présente invention, la composition peut comprendre en outre au moins un autre agent émulsionnant classique, notamment la cyclodiméthicone.

Dans un mode de réalisation particulier de la composition selon l'invention, la proportion d'agent émulsionnant est comprise entre environ 5 et environ 30 % en poids, par rapport au poids total de la composition.

Parmi les dioxydes de titane utilisés comme agent écran inorganique, on peut citer comme dioxyde hydrophobe le Titanium dioxide T 805, comme dioxyde hydrophile dopé en fer le Titanium dioxide PF 2 et comme dioxyde hydrophobe dopé en fer le Titanium dioxide T 817, de la société DEGUSSA. Les oxydes de titane mis en oeuvre dans la présente invention peuvent également être synthétisés selon le procédé AEROSIL® développé par la société DEGUSSA. Ce procédé implique notamment la co-calcination des chlorures de titane et de fer, respectivement TiCL₄ et FeCl₃, à haute température et en présence d'une flamme oxohydrogénée. Les oxydes de titane synthétisés, dopés au fer, présentent une surface BET moyenne de 50m²/g et une taille moyenne de particule de 21 nm.

Aussi, il a été démontré que la capacité d'absorption des UVA et UVB des oxydes de titane augmentait avec leur dispersion. L'introduction de fer au cours du processus de synthèse conduit à la formation d'oxydes mixtes fer-titane, dans lesquels l'oxyde de titane est plus dispersé. Enfin, l'insertion de fer au sein d'une phase oxyde de titane, majoritairement de type anatase, entraîne une modification des propriétés semi-conductrices de l'oxyde de titane et donc de ces propriétés photochimiques.

Selon la présente invention, la phase grasse peut comprendre également d'autres corps gras cosmétiquement ou dermatologiquement acceptables, notamment des huiles animales, végétales ou minérales et leurs analogues, des benzoates d'alcools gras et des triglycérides d'acides gras.

Selon la présente invention, la phase aqueuse comprend de l'eau et des composés hydrophiles cosmétiquement ou dermatologiquement acceptables, parmi lesquels on peut citer la glycérine, et éventuellement des solvants organiques, comme les alcools inférieurs hydrosolubles.

La composition cosmétique ou dermatologique selon l'invention présente une viscosité inférieure à 100 Pa.s (100 000 centipoises) à 25 ° C, mesurée avec un viscosimètre de Brookfield.

Concernant l'altération de l'ADN des cellules de la peau par les rayons UV, son indispensable réparation, qui limitera la pérennisation des dégâts, est contrôlée par la protéine P53 qui ordonne la réparation ou la destruction cellulaire (apoptose). Au cours de l'exposition solaire, la protéine P53 peut devenir inefficace par mutation au niveau du gène d'où prolifération des cellules anormales dont la reconnaissance et le contrôle sont assurés par le système immunitaire (cellule de Langerhans), dernier rempart avant le processus tumoral.

Concernant la photo-immunosuppression, des récentes études ont montré que les rayons UV altèrent les cellules essentielles de l'immunité cutanée, les cellules de Langerhans. Ils les détruisent ou créent des dysfonctionnement en inhibant leur action de reconnaissance et de protection contre les agents étrangers (bactéries, virus, tumeurs, allergènes).

Ces deux agressions en profondeur de la peau, dues à l'action des rayons UV peuvent aboutir à la formation des cancers cutanés.

Or, si la plupart des produits solaires protègent plus ou moins bien contre les rayons UV, en prévenant les coups de soleil, les travaux les plus récents suggèrent que leur efficacité en particulier contre la photo-immunosuppression induite par les rayons UV n'est que partielle et donc une immunosuppression chronique peut se développer en l'absence de coups de soleil visibles et malgré l'application de ces produits solaires.

Certaines études épidémiologiques ont même montré que le risque de cancers cutanés est plus élevé chez les utilisateurs d'écrans solaires, paradoxe qui s'expliquerait par le fait que l'absence de coups de soleil permettraient des expositions plus prolongées et donc favoriseraient les dommages invisibles des rayonnements UV.

Aussi, la composition selon l'invention peut comprendre en outre au moins un agent protecteur contre l'immuno-suppression induite par les rayons ultraviolets, choisi dans le groupe constitué par l'Aloe vera (extrait d'Aloe barbadensis), la vitamine E, l'insaponifiable d'huile de soja et les mélanges de ces derniers, dans une proportion comprise avantageusement entre environ 0,05 % et environ 5 % en poids, par rapport au poids total de la composition.

La composition cosmétique ou dermatologique selon l'invention peut comprendre en outre au moins un agent protecteur de l'ADN des cellules de la peau, choisi dans le groupe constitué par les isoflavones et/ou les sels de zinc, dans une proportion avantageusement comprise entre environ 0,01 % et environ 1 % en poids, par rapport au poids total de la composition, ledit sel étant avantageusement le gluconate de zinc.

Les « isoflavones » utilisables selon ce mode particulier de la présente invention sont obtenues par synthèse chimique ou sont des substances naturelles extraites de produits naturels, notamment à partir de végétaux tels que le soja, le trèfle, le lupin, les pépins de pommme etc. Bien souvent les compositions topiques selon la présente invention contiennent, à titre d'isoflavones un mélange de différentes isoflavones, mais elles peuvent également être présentes sous forme pure dans le cadre de la présente invention. Par ailleurs, on distingue les formes aglycones des isoflavones et les formes glycosylées de ces dernières. Ces diverses formes se trouvent le plus souvent en mélange. Elles sont illustrées par les formules suivantes. Formes aglycones, de formule : 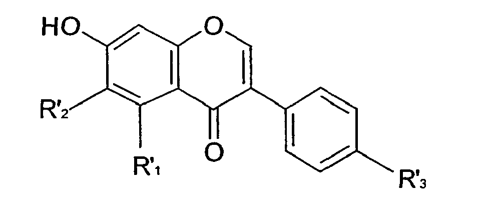 dans laquelle R'₁ représente un atome d'hydrogène ou un groupe hydroxy, R'₂ représente un atome d'hydrogène ou un groupe méthoxy et R'₃ représente un groupe hydroxy.

Avantageusement, selon la présente invention, R'₁, R'₂ et R'₃ représentent :

| **R'₁** | **R'₂** | **R'₃** | **Nom du composé** |
|---|---|---|---|
| H | H | OH | Daidzéine |
| OH | H | OH | Génistéine |
| H | OCH₃ | OH | Glycitéine |

Formes glycosylées, de formule : 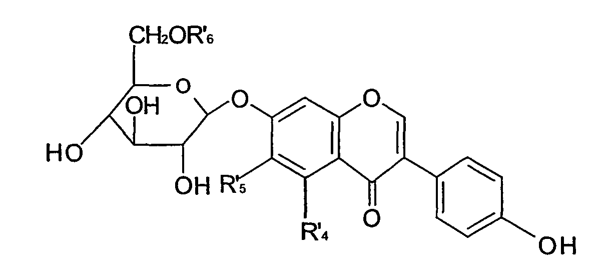 dans laquelle R'₄ représente un atome d'hydrogène ou un groupe hydroxy, R'₅ représente un atome d'hydrogène ou un groupe méthoxy et R'₆ représente un atome d'hydrogène.

Avantageusement, selon la présente invention R'₄, R'₅ et R'₆ représentent :

| **R'₄** | **R'₅** | **R'₆** | **Nom du composé** |
|---|---|---|---|
| H | H | H | Daidzine |
| OH | H | H | Génistine |
| H | OCH₃ | H | Glycitine |

Les formes glycosylées des isoflavones sont les plus abondantes dans la nature.

On préfère, à titre d'isoflavones, les isoflavones naturelles telles que la génistéine (1), la daidzéine ou la glycitéine. 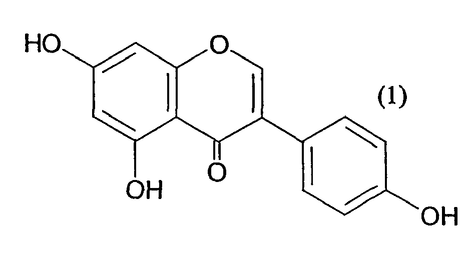 En particulier, la génistéine ou 4,5,7-trihydroxyisoflavone utilisable selon la présente invention peut être un produit d'origine végétale et notamment de soja, titrant 85 à 90 % en poids de génistéine, notamment le produit commercialisé par la société Buckton Scott sous le nom "génistéine titrée à 85%".

Ainsi, la composition cosmétique ou dermatologique selon l'invention présente également, en plus de cette photo-protection à spectre large obtenue par l'association spécifique agent écran minéral - agent émulsionnant, l'avantage d'un effet protecteur "en profondeur" contre l'altération de l'ADN des cellules de la peau et plus particulièrement contre le phénomène de photo-immunosupression.

Bien entendu, la composition selon l'invention peut également contenir un ou plusieurs adjuvants cosmétiques, lipophiles ou hydrophiles, classiques, notamment ceux qui sont déjà utilisés de manière habituelle dans la fabrication et l'obtention des compositions cosmétiques ou dermatologiques solaires.

Ainsi la composition cosmétique ou dermatologique selon l'invention peut comprendre en outre au moins un adjuvant choisi dans le groupe constitué par les épaississants ioniques ou non ioniques, les adoucissants, les antioxydants, les opacifiants, les stabilisants, les émollients, les agents répulsifs contre les insectes, les filtres solaires organiques actifs dans l'UV-A ou l'UV-B (notamment lorsqu'on souhaite l'utiliser chez l'adulte), les agents hydratants, les vitamines, les parfums, les conservateurs, les charges, les séquestrants, les colorants et les mélanges de ces composés.

Il a été observé que la composition cosmétologique ou dermatologique selon l'invention voit son indice de protection (IP) augmenter au cours du stockage, passant de 40 à 70 ou de 20 à 30 après deux mois de stockage, ce qui pourrait présenter un avantage certain lors de son utilisation.

La composition cosmétologique ou dermatologique selon l'invention peut être préparée par toute méthode connue de l'homme du métier, notamment par mélange des différents ingrédients.

La composition cosmétique ou dermatologique selon l'invention peut se présenter sous forme d'une crème, d'un lait, d'un gel, d'un gel-crème, d'un stick pour les lèvres, ou sous toutes autres formes généralement utilisées en cosmétique ou dermatologie pour l'application topique d'une émulsion eau-dans-huile, en particulier celles convenant habituellement aux compositions cosmétiques ou dermatologiques solaires.

Un autre objet de la présente invention réside dans un procédé de traitement cosmétique ou dermatologique de la peau destiné à protéger la peau contre la nocivité et l'agression des ultra-violets et qui consiste essentiellement à appliquer sur celle-ci une quantité efficace d'une composition cosmétique ou dermatologique telle que définie ci-dessus. Les compositions selon l'invention peuvent donc être utilisées dans le traitement des pathologies liées aux UVA et aux UVB, notamment les érythèmes, l'acné, le vieillissement, l'immunosupression, l'inflammation ainsi que l'aggravation d'autres pathologies dermatologiques (acné, rosacée...).

Les exemples qui suivent illustrent l'invention sans toutefois la limiter à ces modes particuliers de réalisation.

Sauf indications contraires, les pourcentages indiqués dans les exemples qui suivent sont des pourcentages en poids total de la composition.

### Exemple 1 : Mode opératoire pour produits solaires

### 1 - PREPARATION DE LA PHASE GRASSE

Dans un réacteur, on introduit la phase grasse (pigments minéraux, émulsionnant et huile) et on homogénéise avec recyclage pour obtenir une bonne dispersion. On chauffe cette phase jusqu'à 60°C.

### 2 - PREPARATION DE LA PHASE AQUEUSE

Dans un conge, sous agitation, on disperse le gélifiant dans la phase aqueuse contenant les électrolytes, puis on ajoute les actifs. On chauffe cette phase jusqu'à 60°C.

### 3 - MISE EN EMULSION

A 60°C, on coule la phase aqueuse lentement (30 min) dans la phase huileuse, en maintenant une agitation assez soutenue (à l'aide d'une turbine ou d'un racleur) et on vérifie l'aspect de l'émulsion.
On homogénéise pendant 30 min sous recyclage en refroidissant le produit jusqu'à 25°C.

On vidange, puis on contrôle le produit après 1 heure de repos pour obtenir une indication de sa viscosité. On vérifie la parfaite régularité de l'émulsion.

### 4 - MESURE DE LA VISCOSITE

La viscosité est mesurée à 25°C avec un viscosimètre de Brookfiled.

| Emulsionnant | IP | Viscosité à 25 °C Pa.s (cp) |
|---|---|---|
| ETA (Cétyldiméthicone polyols) | 35 | 200 (200 000) |
| ETA (Cétyldiméthicone polyols) | 20 | 60 (60 000) |
| Invention (exemple 2) | 70 | 100 (100 000) |
| Invention (exemple 3) | 50 | 60 (60 000) |
| Invention....(exemple 4) | 25 | 15 (15 000) |

Ainsi pour un indice de protection (IP) voisin, les compositions selon l'invention possèdent une viscosité nettement inférieure à celles des compositions de l'état antérieur de la technique (ETA).

### Exemple 2 : Formule avec un indice de protection supérieur à 70

| | |
|---|---|
| Neopentapate d'octyldodécyle | 15 à 30 |
| Dioxide de titane | 15 à 30 |
| Cyclomethicone | 10 à 20 |
| Eau | 5 à 20 |
| Oxyde de zinc | 3 à 10 |
| Ethylhexyl dimethicone Ethoxy Glucoside | 1 à 10 |
| Triglyceride Caprylique/Caprique | 1 à 10 |
| Glycérine | 1 à 10 |
| PEG45/Dodecyl Glycol Copolymère | 1 à 10 |
| Cyclopentasiloxane | 1 à 10 |
| Chlorure de sodium | 1 à 5 |
| Huile insaponifiable de soja | 1 à 5 |
| Palmitate de dextrine | 1 à 5 |
| Hectorite de Stearalkonium | 1 à 5 |
| Phenoxyéthanol | 0,5 |
| Extrait *d'Aloe barbadensis* | 0,1 à 10 |
| Gluconate de zinc | 0,01 à 10 |
| Butylparaben | 0,06 |
| Ethylparaben | 0,04 |
| Propylparaben | 0,02 |

### Exemple 3 : Formule avec un indice de protection supérieur à 50

| | |
|---|---|
| Eau | 20 à 50 |
| Neopentapate d'octyldodécyle | 15 à 40 |
| Dioxide de titane | 10 à 20 |
| Cyclométhicone | 5 à 20 |
| Oxide de zinc | 3 à 15 |
| Glycérine | 3 à 15 |
| Ethylhexyl dimethicone Ethoxy Glucoside | 3 à 15 |
| PEG-45/Dodecyl Glycol Copolymère | 3 à 15 |
| Palmitate de dextrine | 1 à 5 |
| Cyclopentasiloxane | 1 à 5 |
| Chlorure de sodium | 1 à 5 |
| Huile insaponifiable de soja | 1 à 5 |
| Phenoxyéthanol | 0,5 |
| Extrait d'*Aloe barbadensis* | 0,2 |
| Methylparaben | 0,1 à 10 |
| Gluconate de zinc | 0,08 |
| Butylparaben | 0,01 à 10 |
| Ethylparaben | 0,04 |
| Propylparaben | 0,02 |

### Exemple 4 : Formule avec un indice de protection supérieur à 25

| | |
|---|---|
| Eau | 20 à 60 |
| Tetraoctanoate de Pentaerythrityle | 15 à 30 |
| Dioxide de titane | 1 à 10 |
| Cyclomethicone | 1 à 10 |
| Oxyde de zinc | 1 à 10 |
| Benzoate de alkyle C12-15 | 1 à 10 |
| Glycérine | 1 à 10 |
| Ether de Dicaprylyle | 1 à 10 |
| Cyclopentasiloxane | 1 à 10 |
| Ethylhexyl dimethicone Ethoxy Glucoside | 1 à 10 |
| Propylene Glycol Dioctanoate | 1 à 10 |
| Chlorure de sodium | 1 à 5 |
| PEG-45/Dodecyl Glycol Copolymère | 1 à 5 |
| PEG-30 Dipolyhydroxystearate | 1 à 5 |
| Huile insaponifiable de soja | 1 à 5 |
| Palmitate de dextrine | 1 à 5 |
| Phenoxyéthanol | 0,5 |
| Extrait *d'Aloe barbadensis* | 0,1 à 10 |
| Methylparaben | 0,16 |
| Gluconate de zinc | 0,01 à 10 |
| Butylparaben | 0,06 |
| Ethylparaben | 0,04 |
| Propylparaben | 0,02 |

La somme des teneurs en dioxyde de titane et en oxyde de fer étant supérieure ou égale à 6%

### Exemple 5 : Formule avec un indice de protection supérieur à 70

| **Ingrédients** | **% en poids** |
|---|---|
| Neopentanoate d'octyldodécyle | 15 à 30 |
| Dioxyde de titane | 15 à 30 |
| Cyclométhicone | 10 à 20 |
| Eau | 5 à 20 |
| Oxyde de zinc | 3 à 10 |
| Ethylhexyl dimethicone Ethoxy Glucoside | 1 à 10 |
| 2-Heptadecadiènylfurane | 0,1 à 10 |
| 4,5,7-Trihydroxyisoflavone | 0,01 à 10 |
| Triglyceride Caprylique/caprique | 1 à 10 |
| Glycérine | 1 à 10 |
| PEG-45/Dodecyl Glycol Copolymère | 1 à 10 |
| Cyclopentasiloxane | 1 à 10 |
| Chlorure de sodium | 1 à 5 |
| Insaponifiable d'huile de soja | 1 à 5 |
| Palmitate de dextrine | 1 à 5 |
| Hectorite de stéaralkonium | 1 à 5 |
| Phenoxyéthanol | 0,5 |
| Extrait d'*aloe barbadensis* | 0,2 |
| Gluconate dezinc | 0,08 |
| Butylparaben | 0,06 |
| Ethylparaben | 0,04 |
| Propylparaben | 0,02 |

### Exemple 6 : Formule avec un indice de protection supérieur à 50

| **Ingrédients** | **% en poids** |
|---|---|
| Eau | 20 à 50 |
| Neopentanoate d'octyldodécyle | 15 à 40 |
| Dioxyde de titane | 10 à 20 |
| 2-Heptadecadiènylfurane | 0,1 à 10 |
| 4,5,7-Trihydroxyisoflavone | 0,01 à 10 |
| Cyclométhicone | 5 à 20 |
| Oxyde de zinc | 3 à 15 |
| Glycérine | 3 à 15 |
| Ethylhexyl dimethicone Ethoxy Glucoside | 3 à 15 |
| PEG-45/Dodecyl Glycol Copolymer | 3 à 15 |
| Palmitate de dextrine | 1 à 5 |
| Cyclopentasiloxane | 1 à 5 |
| Chlorure de sodium | 1 à 5 |
| Huile d'insaponifiables de soja (Glycine Soja) | 1 à 5 |
| Phénoxyéthanol | 0,5 |
| Extrait *d'Aloe barbadensis* | 0,2 |
| Méthylparaben | 0,16 |
| Gluconate de zinc | 0,08 |
| Butylparaben | 0,06 |
| Ethylparaben | 0,04 |
| Propylparaben | 0,02 |

### Exemple 7 : Formule avec un indice de protection supérieur à 25

| | |
|---|---|
| **Ingrédients** | **% en poids** |
| Eau | 20 à 60 |
| Tetraoctanoate de Pentaerythrityle | 15 à 30 |
| Dioxyde de titane | 1 à 10 |
| Cyclométhicone | 1 à 10 |
| Oxyde de zinc | 1 à 10 |
| Benzoate de (C12-C15)alkyle | 1 à 10 |
| 2-Heptadecadiènylfurane | 0,1 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl dimethicone Ethoxy Glucoside | 1 à 10 |
| Propylene Glycol Dioctanoate | 1 à 10 |
| Chlorure de sodium | 1 à 5 |
| PEG-45/Dodecyl Glycol Copolymer | 1 à 5 |
| PEG-30 Dipolyhydroxystéarate | 1 à 5 |
| Huile d'insaponifiable de soja | 1 à 5 |
| Palmitate de dextrine | 1 à 5 |
| Phénoxyéthanol | 0,5 |
| Extrait d'*aloe barbadensis* | 0,2 |
| Méthylparaben | 0,16 |
| Gluconate de zinc | 0,08 |
| Butylparaben | 0,06 |
| Ethylparaben | 0,04 |
| Propylparaben | 0,02 |

### Exemple 8: Méthode de détermination du facteur de protection solaire

Le niveau de protection solaire fait intervenir la réponse érythémale de la peau aux radiations ultraviolettes. Elle est exprimée par le facteur de protection solaire (FPS) qui est le rapport des énergies nécessaires pour induire une réponse érythémale minimale sur la peau de sujets volontaires protégée ou non par le produit à tester, à l'aide du rayonnement ultra-violet généralement fourni par une source artificielle.

La méthode utilisée est celle du COLIPA (European Cosmetic Toilettery And Perfumes Association) décrite dans « La méthode de détermination du facteur de protection solaire, Ref :94/289, octobre 1994).

On détermine chez chaque volontaire la plus faible dose qui produit un érythème, appelée dose érythémale minimale (DEM), soit sans protection (DEMn), soit avec protection (DEMp) et on calcule FPS comme étant le rapport DEMp/DEMn.

Les compositions selon l'invention ont des indices de protection pouvant atteindre 70.

## Revendications

1. Composition cosmétique ou dermatologique pour la protection contre les rayons ultraviolets, à base d'agent écran inorganique, **caractérisée en ce qu'**elle comprend une émulsion eau-dans-huile contenant une association d'un agent écran inorganique particulaire, constitué par un mélange de dioxyde de titane dopé en fer ou de dioxyde de titane hydrophobe et d'oxyde de zinc, et d'au moins un agent émulsionnant choisi dans le groupe constitué par les dérivés siliconés à composante glucosique comprenant un nombre de motifs glucose compris entre 2 et 10, l'agent écran inorganique particulaire étant dispersé de manière homogène dans l'émulsion eau-dans-huile et sa taille moyenne de particule étant comprise entre 1 et 100 nanomètres, et l'agent écran inorganique particulaire étant présent à hauteur de 6 à 40% en poids.

2. Composition cosmétique ou dermatologique selon la revendication 1 **caractérisée en ce que** le dérivé siliconé est choisi parmi les (C₂-C₃₀)alkylsilicones et les amino (C₂-C₃₀)alkylsilicones.

3. Composition cosmétique ou dermatologique selon la revendication 2, **caractérisée en ce que** le dérivé siliconé à composante glucosique est l'éthylhexyl diméthicone éthoxy glucoside.

4. Composition cosmétique ou dermatologique selon l'une quelconque des revendications précédentes, **caractérisée en ce que** la composition cosmétique comprend en outre de la cyclodiméthicone.

5. Composition cosmétique ou dermatologique selon l'une quelconque des revendications précédentes, **caractérisée en ce que** la proportion de l'agent émulsionnant est comprise entre environ 5 et environ 30 % en poids, par rapport au poids total de la composition.

6. Composition cosmétique ou dermatologique selon l'une quelconque des revendications précédentes, **caractérisée en ce que** le dioxyde titane est du dioxyde de titane dopé au fer.

7. Composition cosmétique ou dermatologique selon l'une quelconque des revendications précédentes, **caractérisée en ce que** le dioxyde de titane est du dioxyde de titane hydrophobe.

8. Composition cosmétique ou dermatologique selon l'une quelconque des revendications précédentes, **caractérisée en ce qu'**elle présente une viscosité inférieure à 100 Pa.s (100 000 centipoises) à 25 ° C.

9. Composition cosmétique ou dermatologique selon l'une quelconque des revendications précédentes, **caractérisée en ce qu'**elle comprend en outre au moins un agent protecteur contre l'immuno-suppression induite par les rayons ultraviolets, choisi dans le groupe constitué par l'Aloe vera, la vitamine E, l'insaponifiable d'huile de soja et les mélanges de ces derniers.

10. Composition cosmétique ou dermatologique selon la revendication 9, **caractérisée en ce que** la proportion de l'agent protecteur contre l'immunosuppression induite par les rayons ultraviolets est comprise entre environ 0,05 et environ 5 % en poids, par rapport au poids total de la composition.

11. Composition cosmétique ou dermatologique selon l'une quelconque des revendications précédentes, **caractérisée en ce qu'**elle comprend en outre au moins un agent protecteur de l'ADN des cellules de la peau, choisi dans le groupe constitué par les isoflavones et/ou les sels de zinc.

12. Composition cosmétique ou dermatologique selon la revendication 11, **caractérisée en ce que** l'agent protecteur des cellules de la peau est le gluconate de zinc.

13. Composition cosmétique ou dermatologique selon la revendication 11 ou 12, **caractérisée en ce que** la proportion de l'agent protecteur des cellules de la peau est comprise entre environ 0,01 et environ 1 % en poids, par rapport au poids total de la composition.

14. Composition cosmétique ou dermatologique selon l'une quelconque des revendications précédentes, **caractérisée en ce qu'**elle comprend en outre au moins un adjuvant choisi dans le groupe constitué par les épaississants ioniques ou non ioniques, les adoucissants, les antioxydants, les opacifiants, les stabilisants, les émollients, les agents répulsifs contre les insectes, les filtres solaires organiques actifs dans l'UV-A ou l'UV-B, les agents hydratants, les vitamines, les parfums, les conservateurs, les charges, les séquestrants, les colorants et les mélanges de ces derniers.

15. Utilisation des compositions selon l'une quelconque des revendications 1 à 14 pour la fabrication de compositions destinées à la protéger la peau contre la nocivité et l'agression des ultra-violets.

16. Procédé de traitement cosmétique de la peau destiné à protéger la peau contre la nocivité et l'agression des ultra-violets et qui consiste à appliquer sur celle-ci une quantité efficace d'une composition cosmétique telle que définie à l'une quelconque des revendications 1 à 14.

## Claims

1. Cosmetic or dermatological composition for protecting against ultraviolet radiation, based on an inorganic screening agent, **characterized in that** it comprises a water-in-oil emulsion containing a combination of a particulate inorganic screening agent, consisting of a mixture of titanium dioxide doped with iron or of hydrophobic titanium dioxide and of zinc oxide, and at least one emulsifying agent chosen from the group consisting of silicone derivatives with a glycosidic constituent comprising a number of glucose units of between 2 and 10, the particulate inorganic screening agent being homogeneously dispersed in the water-in-oil emulsion and its mean particle size being between 1 and 100 nanometers, and the particulate inorganic screening agent being present in an amount of 6 to 40% by weight.

2. Cosmetic or dermatological composition according to Claim 1, **characterized in that** the silicone derivative is chosen from (C₂-C₃₀)alkylsilicones and amino(C₂-C₃₀)alkylsilicones.

3. Cosmetic or dermatological composition according to Claim 2, **characterized in that** the silicone derivative with a glycosidic constituent is ethylhexyl dimethicone ethoxy glucoside.

4. Cosmetic or dermatological composition according to any one of the preceding claims, **characterized in that** the cosmetic composition comprises, in addition, cyclodimethicone.

5. Cosmetic or dermatological composition according to any one of the preceding claims, **characterized in that** the proportion of emulsifying agent is between about 5 and about 30% by weight, relative to the total weight of the composition.

6. Cosmetic or dermatological composition according to any one of the preceding claims, **characterized in that** the titanium dioxide is titanium dioxide doped with iron.

7. Cosmetic or dermatological composition according to any one of the preceding claims, **characterized in that** the titanium dioxide is hydrophobic titanium dioxide.

8. Cosmetic or dermatological composition according to any one of the preceding claims, **characterized in that** it has a viscosity of less than 100 Pa.s (100 000 centipoises) at 25°C.

9. Cosmetic or dermatological composition according to any one of the preceding claims, **characterized in that** it comprises, in addition, at least one agent protecting against the immunosuppression induced by ultraviolet radiation, chosen from the group consisting of Aloe vera, vitamin E, the unsaponifiable component of soybean oil and mixtures thereof.

10. Cosmetic or dermatological composition according to Claim 9, **characterized in that** the proportion of agent protecting against the immunosuppression induced by ultraviolet radiation is between about 0.05 and about 5% by weight, relative to the total weight of the composition.

11. Cosmetic or dermatological composition according to any one of the preceding claims, **characterized in that** it comprises, in addition, at least one agent protecting the DNA of skin cells, chosen from the group consisting of isoflavones and/or zinc salts.

12. Cosmetic or dermatological composition according to Claim 11, **characterized in that** the agent protecting the skin cells is zinc gluconate.

13. Cosmetic or dermatological composition according to Claim 11 or 12, **characterized in that** the proportion of agent protecting the skin cells is between about 0.01 and about 1% by weight, relative to the total weight of the composition.

14. Cosmetic or dermatological composition according to any one of the preceding claims, **characterized in that** it comprises, in addition, at least one adjuvant chosen from the group consisting of ionic or nonionic thickeners, demulcents, antioxidants, opacifiers, stabilizers, emollients, insect repellents, organic sunscreens which are active in UV-A or UV-B, moisturizers, vitamins, perfumes, preservatives, fillers, sequestrants, colorants and mixtures of these compounds.

15. Use of the compositions according to any one of Claims 1 to 14 for the manufacture of compositions intended for protecting the skin against the harmfulness of and attack by ultraviolet radiation.

16. Method for the cosmetic treatment of the skin intended to protect the skin against the harmfulness of and attack by ultraviolet radiation and which consists in applying thereto an effective quantity of a cosmetic composition as defined in any one of Claims 1 to 14.

## Patentansprüche

1. Kosmetische oder dermatologische Zusammensetzung zum Schutz gegen Ultraviolettstrahlen auf der Basis eines anorganischen Abschirmungsmittels, **dadurch gekennzeichnet, dass** sie eine Wasser-in-Öl-Emulsion umfasst, die eine Assoziation eines anorganischen teilchenförmigen Abschirmungsmittels, welches aus einer Mischung von mit Eisen dotiertem Titandioxid oder hydrophobem Titandioxid und Zinkoxid besteht, und mindestens eines Emulgiermittels enthält, das ausgewählt ist aus der Gruppe bestehend aus siliconhaltigen Derivaten mit Glucose-Komponente, welche eine Zahl von Glucose-Motiven zwischen zwei und zehn einschließlich umfasst, wobei das teilchenförmige anorganische Abschirmungsmittel auf homogene Weise in der Wasser-in-Öl-Emulsion dispergiert ist und seine mittlere Teilchengröße zwischen 1 und 100 Nanometern einschließlich liegt und wobei das teilchenförmige anorganische Abschirmungsmittel in einer Menge von 6 bis 40 Gew.-% vorliegt.

2. Kosmetische oder dermatologische Zusammensetzung nach Anspruch 1, **dadurch gekennzeichnet, dass** das siliconhaltige Derivat aus (C₂-C₃₀)-Alkylsiliconen und Amino-(C₂-C₃₀)-alkylsiliconen ausgewählt ist.

3. Kosmetische oder dermatologische Zusammensetzung nach Anspruch 2, **dadurch gekennzeichnet, dass** das siliconhaltige Derivat mit Glucose-Komponente Ethylhexyldimethiconethoxyglucosid ist.

4. Kosmetische oder dermatologische Zusammensetzung nach irgendeinem der vorangehenden Ansprüche, **dadurch gekennzeichnet, dass** die kosmetische Zusammensetzung darüber hinaus Cyclodimethicon umfasst.

5. Kosmetische oder dermatologische Zusammensetzung nach irgendeinem der vorangehenden Ansprüche, **dadurch gekennzeichnet, dass** der Prozentsatz des Emulgiermittels zwischen etwa 5 und etwa 30 Gew.-%, bezogen auf das Gesamtgewicht der Zusammensetzung, beträgt.

6. Kosmetische oder dermatologische Zusammensetzung nach irgendeinem der vorangehenden Ansprüche, **dadurch gekennzeichnet, dass** das Titandioxid mit Eisen dotiertes Titandioxid ist.

7. Kosmetische oder dermatologische Zusammensetzung nach irgendeinem der vorangehenden Ansprüche, **dadurch gekennzeichnet, dass** das Titandioxid hydrophobes Titandioxid ist.

8. Kosmetische oder dermatologische Zusammensetzung nach irgendeinem der vorangehenden Ansprüche, **dadurch gekennzeichnet, dass** sie eine Viskosität von weniger als 100 Pa.s (100 000 Centipoise) bei 25 °C aufweist.

9. Kosmetische oder dermatologische Zusammensetzung nach irgendeinem der vorangehenden Ansprüche, **dadurch gekennzeichnet, dass** sie darüber hinaus mindestens ein Schutzmittel gegen die Immunsuppression umfasst, die durch Ultraviolettstrahlen induziert wird, welches ausgewählt ist aus der Gruppe bestehend aus Aloe vera, Vitamin E, den unverseifbaren Teilen von Sojaöl und Mischungen der letztgenannten.

10. Kosmetische oder dermatologische Zusammensetzung nach Anspruch 9, **dadurch gekennzeichnet, dass** der Prozentsatz des Schutzmittels gegen die Immunsuppression, die von Ultraviolettstrahlen induziert wird, zwischen etwa 0,05 und etwa 5 Gew.-%, bezogen auf das Gesamtgewicht der Zusammensetzung, beträgt.

11. Kosmetische oder dermatologische Zusammensetzung nach irgendeinem der vorangehenden Ansprüche, **dadurch gekennzeichnet, dass** sie darüber hinaus mindestens ein Schutzmittel für die DNA der Hautzellen umfasst, das ausgewählt ist aus der Gruppe bestehend aus Isoflavonen und/oder Zinksalzen.

12. Kosmetische oder dermatologische Zusammensetzung nach Anspruch 11, **dadurch gekennzeichnet, dass** das Schutzmittel für die Hautzellen Zinkgluconat ist.

13. Kosmetische oder dermatologische Zusammensetzung nach Anspruch 11 oder 12, **dadurch gekennzeichnet, dass** der Prozentsatz des Schutzmittels für Hautzellen zwischen etwa 0,01 und etwa 1 Gew.-%, bezogen auf das Gesamtgewicht der Zusammensetzung, beträgt.

14. Kosmetische oder dermatologische Zusammensetzung nach irgendeinem der vorangehenden Ansprüche, **dadurch gekennzeichnet, dass** sie darüber hinaus mindestens ein Hilfsmittel enthält, das ausgewählt ist aus der Gruppe bestehend aus ionischen oder nichtionischen Verdickungsmitteln, reizmindernden Mitteln, Antioxidanzien, Trübungsmitteln, Stabilisatoren, Hautgeschmeidigern, Abwehrmitteln gegen Insekten, organischen Sonnenfiltern, die in UV-A oder UV-B aktiv sind, Befeuchtungsmitteln, Vitaminen, Parfums, Konservierungsmitteln, Füllstoffen, Komplexbildnern, Farbstoffen und Mischungen der letztgenannten.

15. Verwendung von Zusammensetzungen nach irgendeinem der Ansprüche 1 bis 14 für die Herstellung von Zusammensetzungen, die dazu bestimmt sind, die Haut vor der Schädlichkeit und dem Angriff von Ultraviolettstrahlen zu schützen.

16. Verfahren zur kosmetischen Behandlung der Haut, das dazu bestimmt ist, die Haut vor der Schädlichkeit und dem Angriff von Ultraviolettstrahlen zu schützen, und das darin besteht, auf diese eine wirksame Menge einer kosmetischen Zusammensetzung, wie in irgendeinem der Ansprüche 1 bis 14 definiert, aufzutragen.
